# EUROPEAN PATENT APPLICATION

(11) **EP 2 014 639 A1**
(43) Date of publication of application: **14.01.2009**
(21) Application number: 07742063.6
(22) Date of filing: 20.04.2007
(51) Int. Cl.: C07C 69/653, C07C 31/44, C07C 67/14, C07D 301/24, C07D 303/30, C08L 101/00, G02B 1/04, G02B 1/11

(54) **FLUORINE-CONTAINING ADAMANTANE DERIVATIVE, FLUORINE-CONTAINING ADAMANTANE DERIVATIVE HAVING POLYMERIZABLE GROUP, RESIN COMPOSITION CONTAINING THE SAME, AND ANTIREFLECTION FILM**

(30) Priority: 28.04.2006 JP 2006125441
(71) Applicant: Idemitsu Kosan Co., Ltd., Chiyoda-ku Tokyo 100-8321 (JP)
(72) Inventor: OKADA, Yasunari, Ichihara-shi, Chiba 299-0193 (JP); YAMANE, Hideki, Ichihara-shi, Chiba 299-0193 (JP); ITO, Hajime, Ichihara-shi, Chiba 299-0193 (JP); MATSUMOTO, Nobuaki, Ichihara-shi, Chiba 299-0193 (JP)
(74) Representative: Vossius & Partner
(86) International application number: PCT/JP2007/058628
(87) International publication number: WO 2007/125829

(57) **Abstract**

To provide a polymerizable group-containing and fluorine-containing adamantane derivative capable of affording a cured product having good heat resistance, good mechanical properties such as mar resistance and a low refractive index, a resin composition containing such a polymerizable group-containing adamantane derivative, and a fluorine-containing adamantane derivative which is useful as a reaction intermediate used for the production of the polymerizable group-containing and fluorine-containing adamantane derivative.

Specifically provided are a fluorine-containing adamantane derivative represented by the general formula (I) below, a polymerizable group-containing and fluorine-containing adamantane derivative represented by the general formula (II) below, and a resin composition containing such a polymerizable group-containing and fluorine-containing adamantane derivative. In the formula, R¹ and R² are each a hydrogen atom, for example, n is an integer of 0 or more, X¹ is a polymerizable group represented by the formula (III-a), for example, Y is a hydrogen atom, for example, s and t are each an integer of 1 to 15, and u is an integer of 0 to 14, with the proviso that s + t + u = 16.

## Description

### TECHNICAL FIELD

The present invention relates to a novel fluorine-containing adamantane derivative, a novel polymerizable group-containing and fluorine-containing adamantane derivative, a method for preparing the same, and a resin composition containing the polymerizable group-containing and fluorine-containing adamantane derivative and a reflection preventing film. More specifically, the present invention is directed to a polymerizable group-containing and fluorine-containing adamantane derivative which is capable of giving a cured product having good heat resistance and good mechanical properties such as mar resistance and having a low refractive index, and which may be used as a reflection preventing film material for a display such as a liquid crystal or an organic EL element, a reflection preventing film material for a semiconductor resist, a refractive index modulation material for a volume hologram, and materials for optical fibers, optical waveguides and various types of lenses, to a resin composition containing the same, to a fluorine-containing adamantane derivative useful as a reaction intermediate used for producing such a polymerizable group-containing and fluorine-containing adamantane derivative, to a method capable of producing such a polymerizable group-containing and fluorine-containing adamantane derivative in an efficient manner, and to a reflection preventing film having a layer obtained by curing the resin composition.

### BACKGROUND ART

Adamantane is a stable, highly symmetrical compound in which four cyclohexane rings are condensed to form a cage-like structure. It is known that adamantane derivatives, which show peculiar functions, are useful as raw materials for medical materials and highly functional industrial materials. Further, because adamantane has specific optical characteristics and heat resistance, an attempt has been made to use it as, for example, an optical disc substrate, an optical fiber or a lens (see, for example, Patent Documents 1 and 2). Another attempt has also been made to use an adamantane ester as a raw material for a resin photoresist by utilizing its sensitivity to an acid, dry etching resistance and transparency to UV rays (see, for example, Patent Document 3).
In recent years, studies have been made to attain high fineness, wide sight angle and high image quality of flat panel displays using liquid crystals or organic electroluminescence (EL) elements, to use high frequency in electronic circuits and to achieve high performance and improvement of optical and electronic parts in optical circuits and optical communication.
In this circumstance, an attempt is being made to improve fluorine-containing material which is used in a low refraction index layer for a reflection preventing film of displays and in optical fibers and optical waveguides for optical communication. Since, in general, a fluorine atom-containing compound has a low refractive index, studies are being made to use a fluorine-containing resin material having a low refractive index for a reflection preventing film for liquid crystals and organic EL displays, a lens such as a Fresnel lens, a lenticular lens or a microlens array, an optical fiber and an optical waveguide. For index and high refractive index layers are alternately laminated for the prevention of reflection, a straight chain polymer of a fluorine-containing acrylate is used as a resin of the low refractive index layer (see, for example, Patent Documents 4 to 6). Because the resin is straight-chained, a sufficiently high surface hardness cannot be obtained and a problem is caused with respect to the mar resistance. In the field of optical fibers and optical waveguides, it is well known that C-H bonds in an organic compound cause an optical loss. To cope with this problem, a material in which such C-H bonds are replaced by C-F bonds is used. For example, the use of a straight chain fluorine-containing acrylate resin is proposed (see, for example, Patent Document 7). The heat resistance of such an acrylate resin, however, is insufficient to withstand reflow soldering and heat generation at the time of communication.

Patent Document 1: Japanese Unexamined Patent Application Publication No. H06-305044
Patent Document 2: Japanese Unexamined Patent Application Publication No. H09-302077
Patent Document 3: Japanese Unexamined Patent Application Publication No. H04-39665
Patent Document 4: Japanese Unexamined Patent Application Publication No. H11-2702
Patent Document 5: Japanese Unexamined Patent Application Publication No. 2001-48943.
Patent Document 6: Japanese Unexamined Patent Application Publication 2004-212619
Patent Document 7: Japanese Unexamined Patent Application Publication 2002-182046

### DISCLOSURE OF THE INVENTION

### Problems to be Solved by the Invention

In view of the foregoing circumstances, the present invention has as its object the provision of a polymerizable group-containing and fluorine-containing adamantane derivative which is capable of giving a cured product having good heat resistance and good mechanical properties such as mar resistance and having a low refractive index, and which may be used as a reflection preventing film material for a display such as a liquid crystal or an organic EL element, a reflection preventing film material for a semiconductor resist, a refractive index modulation material for a volume hologram, and materials for optical fibers, optical waveguides and various types of lenses, of a resin composition containing the same, of a fluorine-containing adamantane derivative useful as a reaction intermediate used for producing such a polymerizable group-containing and fluorine-containing adamantane derivative, of a method capable of producing such a polymerizable group-containing and fluorine-containing adamantane derivative in an efficient manner, and of a reflection preventing film having a layer obtained by curing the resin composition.

### Means for Solving the Problems

The present inventors have made an earnest study and, as a result, have found that a resin composition capable of affording a cured product having good heat resistance and good mechanical properties such as mar resistance and providing a low refractive index may be obtained by using a polymerizable group-containing and fluorine-containing adamantane derivative having a specific structure. It has been also found that a fluorine-containing adamantane derivative having a specific structure is useful as a reaction intermediate for the production of the above-described polymerizable group-containing and fluorine-containing adamantane derivative having a specific structure. The present invention has been completed on the basis of such findings.
Thus, the present invention provides a fluorine-containing adamantane derivative, a polymerizable group-containing and fluorine-containing adamantane derivative, a method for producing same, and a resin composition containing the same and a reflection preventing film, as follows.
1. A fluorine-containing adamantane derivative represented by the following general formula (I):

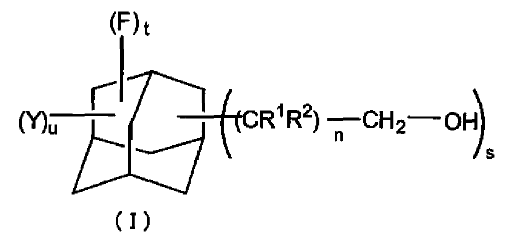

[wherein R¹ and R² each independently represent a hydrogen atom, a halogen atom or a C₁ to C₂₀ aliphatic hydrocarbon group which may contain a heteroatom or heteroatoms, n represents an integer of 0 or more, Y represents a group selected from a hydrogen atom, a hydrocarbon group, a halogen-substituted hydrocarbon group, a cyclic hydrocarbon group, a halogen-substituted cyclic hydrocarbon group, a hydroxyl group, a carboxyl group and =O formed by two Y's taken together, s and t each represent an integer of 1 to 15 and u represents an integer of 0 to 14 with the proviso that a sum of s, t and u is equal to 16].
2. A polymerizable group-containing and fluorine-containing adamantane derivative represented by the following general formula (II):

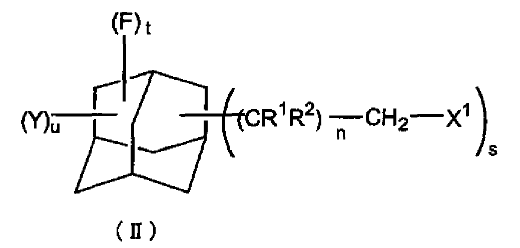

[wherein R¹, R², n, Y, s, t and u are the same as above, X¹ represents a polymerizable group represented by the following general formula (III), the following formula (IV) or the following general formula (V) :

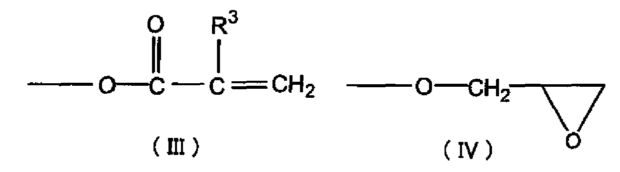

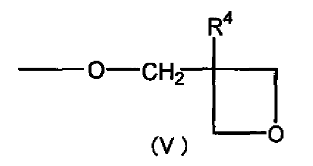

(where R³ represents a hydrogen atom, a methyl group or a trifluoromethyl group and R⁴ represents a C₁ to C₅ hydrocarbon group)
Y represents a group selected from a hydrogen atom, a hydrocarbon group, a halogen-substituted hydrocarbon group, a hydroxyl group, a carboxyl group and =O formed by two Y's taken together, s and
t each represent an integer of 1 to 15 and u represents an integer of 0 to 14 with the proviso that a sum of s, t and u is equal to 16].
3. A process for producing a polymerizable group-containing and fluorine-containing adamantane derivative represented by the general formula (II) shown below, comprising reacting a fluorine containing adamantane derivative represented by the general formula (I) shown below with a compound having a polymerizable group:

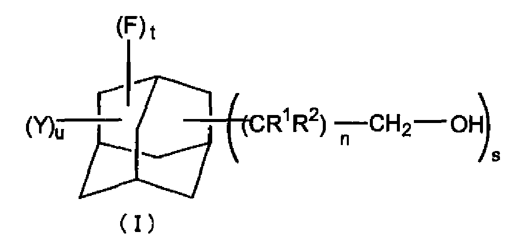

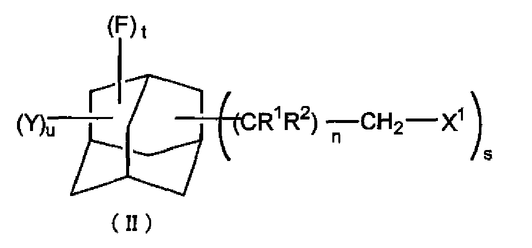

[R¹, R², n, X¹, Y, s, t and u are the same as above]
4. A resin composition comprising a polymerizable group-containing and fluorine-containing adamantane derivative according to above 2.
5. A reflection preventing film having a layer obtained by curing a resin composition according to above 4.

### Effect of the Invention

A polymerizable group-containing and fluorine-containing adamantane derivative and a resin composition containing the adamantane derivative according to the present invention are capable of giving a cured product which has good heat resistance and good mechanical properties such as mar resistance, which provides a low refractive index, and which may be suitably used as a reflection preventing film material for a display such as an organic EL element or a liquid crystal, a reflection preventing film material for a semiconductor resist, a refractive index modulation material for a volume hologram, and materials for optical fibers, optical waveguides and various types of lenses.

### BEST MODE FOR CARRYING OUT THE INVENTION

The fluorine-containing adamantane derivative of the present invention (hereinafter occasionally referred to as "the fluorine-containing adamantane derivative (I)") is represented by the following general formula (I).

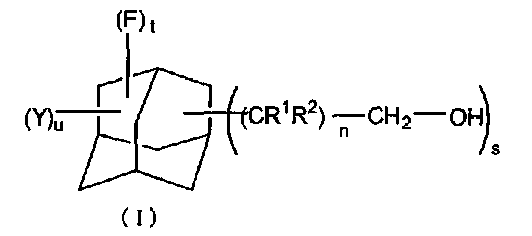

In the above formula, R¹ and R² each independently represent a hydrogen atom, a halogen atom or a C₁ to C₂₀, preferably C₁ to C₁₅, aliphatic hydrocarbon group which may contain a heteroatom or heteroatoms. The halogen atom may be, for example, fluorine, chlorine, bromine or iodine. As the aliphatic hydrocarbon group which may contain a heteroatom or heteroatoms, there may be mentioned, for example, a methoxy group, an ethoxy group, a butoxy group, a hydroxymethyl group, a hydroxyethyl group, a methylthio group, an ethylthio group, a methylamino group, a dimethylamino group, an ethylamino group and a diethylamino group. n is an integer of 0 or more.
In the above general formula (I) , Y represents a hydrogen atom, a hydrocarbon group, a halogen-substituted hydrocarbon group, a cyclic hydrocarbon group, a halogen-substituted cyclic hydrocarbon group, a hydroxyl group or a carboxyl group, or two Y's may be taken together to represent a =O group.

Example of the hydrocarbon group represented by Y include a C₁ to C₁₀ alkyl group and a C₁ to C₁₀ alkoxy group. The alkyl group may be straight chained, branched or cyclic. Specific examples of the alkyl group include a methyl group, an ethyl group, a propyl group, a butyl group or a cylohexyl group. The alkoxy group may be, for example, a methoxy group or an ethoxy group. The halogen-substituted hydrocarbon group may be, for example, a group obtained by replacing at least one of the hydrogen atoms of the above-described hydrocarbon group with a halogen atom or atoms, such as a trifluoromethyl group. The halogen atom may be, for example, fluorine, chlorine, bromine or iodine.
As the cyclic hydrocarbon group represented by Y, there may be mentioned, for example, a C₅ to C₁₀ cycloalkyl group such as a cylopentyl group, a methylcylopentyl group, a cyclohexyl group, a methylcyclohexyl group and an ethylcyclohexyl group. The halogen-substituted cyclic hydrocarbon group may be, for example, a group obtained by replacing at least one of the hydrogen atoms of the above-described cyclic hydrocarbon group with a halogen atom or atoms, such as a fluorocyclopentyl group, a fluorocyclohexyl group, a trifluoromethylcyclopentyl group or a trifluoromethylcyclohexyl group. The symbol s is an integer of 1 to 15, preferably 1 to 12, t is an integer of 1 to 15, preferably 4 to 15, and u is an integer of 0 to 14, preferably 0 to 4, with the proviso that s + t + u = 16.

As the fluorine-containing adamantane derivative I, there may be mentioned, for example, perfluoro-1-adamantanemethanol, perfluoro-2-adamantanemethanol, perfluoro-4-oxo-1-adamantanemethanol, perfluoro-4-oxo-2-adamantanemethanol, perfluoro-1,3-adamantanedimethanol, perfluoro-1,3,5-adamantanetrimethanol, perfluoro-1,3,5,7-adamantanetetramethanol, 2,2-difluoro-2-(perfluoro-1-adamantyl)ethanol, 2,2,3,3-tetrafluoro-3-(perfluoro-1-adamantyl)propane-1-ol, 2,2'-(perfluoroadamantane-1,3-diyl)bis(2,2-difluoroethanol) and 3,3'-(perfluoroadamantane-1,3-diyl)bis(2,2,3,3-tetrafluoropro pane-1-ol).
The polymerizable group-containing and fluorine-containing adamantane derivative (hereinafter also occasionally referred to as "fluorine-containing adamantane derivative (II)") is represented by the following general formula (II).

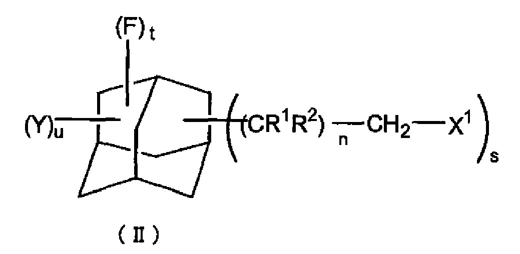

In the formula, R¹, R², n, Y, s, t and u have the same meaning as above, and X¹ represents a polymerizable group represented by the general formula (III), the general formula (IV) or the general formula (V) below.

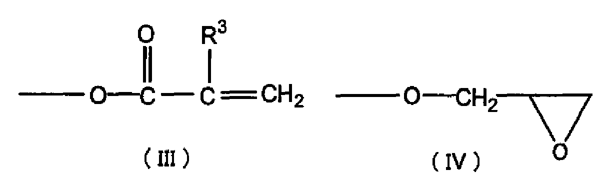

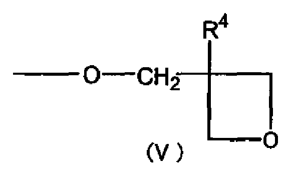

In the above general formula (III), R³ represents a hydrogen atom, a methyl group or a trifluoromethyl group. In the above general formula (V), R⁴ represents a C₁ to C₅ hydrocarbon group.
As the C₁ to C₅ hydrocarbon group, there may be mentioned an alkyl group and an alkoxy group. The alkyl group may be straight chained, branched or cyclic. Specific examples of the alkyl group include a methyl group, an ethyl group, a propyl group and a butyl group.
The alkoxy group may be, for example, a methoxy group or an ethoxy group.

The fluorine-containing adamantane (II) may be prepared by using the above-described fluorine-containing adamantane (I) as a reaction intermediate. Thus, the reaction intermediate is reacted with a polymerizable group-containing compound for conventional esterification or glycidyl etherification.
The polymerizable group-containing compound to be reacted with the above-described fluorine-containing adamantane (I) to form an ester may be, for example, acrylic acid, methacrylic acid, α-trifluoromethylacrylic acid, acrylic chloride, methacrylic chloride and α-trifluoromethylacrylic chloride.

A fluorine-containing adamantane ester derivative as the fluorine-containing adamantane derivative (II) in which X¹ in the general formula (II) is a group represented by the general formula (III) may be prepared by a conventional azeotropic dehydration or acid chloride method. The azeotropic dehydration is generally carried out at a temperature of about 50 to 200°C, preferably 100 to 180°C. A reaction temperature of 50°C or higher can reduce the reaction time because the reaction rate is not lowered and is appropriate. When the reaction temperature is not higher than 200°C, side reactions are prevented from occurring and, further, the product can be prevented from coloring. The reaction pressure in terms of absolute pressure is 0.01 to 10 MPa, preferably between ambient pressure and 1 MPa. When the pressure is 10 MPa or less, it is not necessary to use specific apparatuses because safety is ensured. This is industrially advantageous. The reaction time is generally about 1 to 24 hours, preferably 1 to 10 hours.
The above reaction is performed using a catalyst such as sulfuric acid or p-toluenesulfonic acid. The catalyst is used in an amount of 0.01 to 20 mol %, preferably 0.05 to 10 mol %, based on the fluorine-containing adamantane derivative (I).

The reaction is carried out in the absence or presence of a solvent. It is advantageous that the solvent used can dissolve at least 0.5 % by mass, preferably at least 5 % by mass, of the above-described fluorine-containing adamantane derivative (I). The solvent may be used in such an amount as to provide a concentration of the above-described fluorine-containing adamantane derivative (I) of at least 0.5 % by mass, preferably at least 5 % by mass. The above-described fluorine-containing adamantane derivative (I) may be present in a suspended state but is preferably in a dissolved state. Specific examples of the solvent include nonane, decane, undecane, cyclohexane, methylcyclohexane, toluene, xylene, DMF (dimethylformamide), NMP (N-methyl-2-pyrrolidone), DMAc (N,N-dimethylacetaminde) and DMSO (dimethylsulfoxide). These may be used singly or in combination of two or more thereof.
In performing the above reaction, a polymerization inhibitor such as hydroquinone, methoquinone, phenothiazine or methoxyphenothiazine may be added, if necessary. Such a polymerization inhibitor may be generally used in an amount of about 10 to 10,000 ppm by mass, preferably 50 to 5000 ppm by mass, based on the fluorine-containing adamantane derivative (I).

When the fluorine-containing adamantane derivative (II) is produced by an acid chloride method, the reaction is generally carried out at a temperature of about -50 to 100°C, preferably 0 to 50°C. A reaction temperature of -50°C or higher can reduce the reaction time because the reaction rate is not lowered and is appropriate. When the reaction temperature is not higher than 100°C, side reactions are prevented from occurring and, further, the product can be prevented from coloring. The reaction pressure in terms of absolute pressure is 0.01 to 10 MPa, preferably between ambient pressure and 1 MPa. When the pressure is 10 MPa or less, it is not necessary to use specific apparatuses because safety is ensured. This is industrially advantageous. The reaction time is generally about 5 minute to 10 hours, preferably 1 to 10 hours.
In performing the above reaction, an organic amine, such as triethylamine, tributylamine, pyridine or dimethylaminopyridine, or an inorganic base, such as sodium hydroxide, potassium hydroxide, sodium carbonate, potassium carbonate, sodium phosphate or potassium phosphate, may be added as a scavenger for an acid formed in situ during the reaction. Such a base may be used in such an amount as to provide a molar ratio of the base to the fluorine-containing adamantane derivative (I) of about 0.5 to 5, preferably 1 to 4.

The reaction is carried out in the absence or presence of a solvent. It is advantageous that the solvent used can dissolve at least 0.5 % by mass, preferably at least 5 % by mass, of the above-described fluorine-containing adamantane derivative (I). The solvent may be used in such an amount as to provide a concentration of the above-described fluorine-containing adamantane derivative (I) of at least 0.5 % by mass, preferably at least 5 % by mass. The above-described fluorine-containing adamantane derivative (I) may be present in a suspended state but is preferably in a dissolved state. Specific examples of the solvent include hexane, heptane, cyclohexane, toluene, DMF (dimethylformamide), NMP (N-methyl-2-pyrrolidone), DMAc (N,N-dimethylacetaminde), DMSO (dimethylsulfoxide), ethyl acetate, diethyl ether, tetrahydrofuran, dichloromethane, dichloroethane, dichloroethylene, trichloroethane, trichloroethylene, tetrachloroethane, tetrachloroethylene and chloroform. These may be used singly or in combination of two or more thereof.
In performing the above reaction, a polymerization inhibitor such as hydroquinone, methoquinone, phenothiazine or methoxyphenothiazine may be added, if necessary. Such a polymerization inhibitor may be generally used in an amount of about 10 to 10,000 ppm by mass, preferably 50 to 5000 ppm by mass, based on the fluorine-containing adamantane derivative (I).
In either of the azeotropic dehydration method and the acid chloride method, the obtained reaction product may be refined by distillation, crystallization, column separation, etc. The refining method may be suitably selected depending upon the properties of the product and the kind of impurities.

A fluorine-containing adamantane derivative, which is the fluorine-containing adamantane derivative II having an epoxy group represented by the formula (IV) or an oxetanyl group represented by the general formula (V), may be prepared by reacting the fluorine-containing adamantane derivative I with a compound having an epoxy group or an oxetanyl group. In this case, the compound to be reacted with the fluorine-containing adamantane derivative (I) may be, for example, epichlorohydrin, epibromohydrin, 3-chloromethyl-3-methyloxetane, 3-chloromethyl-3-ethyloxetane, 3-hydroxymethyl-3-methyloxetane or 3-hydroxymethyl-3-ethyloxetane.
The reaction of the above-described compound with the fluorine-containing adamantane derivative (I) is generally carried out at a temperature of about 0 to 200°C, preferably 20 to 150°C. A reaction temperature of 0°C or higher can reduce the reaction time because the reaction rate is not lowered and is appropriate. When the reaction temperature is not higher than 200°C, the product can be prevented from coloring. The reaction pressure in terms of absolute pressure is 0.01 to 10 MPa, preferably between ambient pressure and 1 MPa. When the pressure is 10 MPa or less, it is not necessary to use specific apparatuses because safety is ensured. This is industrially advantageous. The reaction time is generally about 1 minute to 24 hours, preferably 1 to 10 hours.

The above reaction is generally carried out in the presence of a basic catalyst. As the basic catalyst, there may be mentioned, for example, sodium amide, triethylamine, tributylamine, trioctylamine, pyridine, N,N-dimethylaniline, 1,5-diazabicyclo[4.3.0]nonene-5 (DBN), 1,8-diazabicyclo[5.4.0]undecene-7 (DBU), tetramethylammonium chloride, tetraethylammonium chloride, sodium hydroxide, potassium hydroxide, sodium hydride, sodium phosphate or potassium phosphate, sodium carbonate, potassium carbonate, silver oxide, sodium methoxide or potassium t-butoxide.
Such a basic catalyst is used in such an amount as to provide a molar ratio of the basic catalyst to the fluorine-containing adamantane derivative (I) of about 0.5 to 10, preferably 1 to 5.

To the above-described basic catalyst, a quaternary ammonium salt such as tetramethylammonium chloride or tetraethylammonium bromide may be added as a phase transfer catalyst. The quaternary ammonium salt is used in an amount of about 0.01 to 20 mol %, preferably 0.1 to 10 mol %, based on the basic catalyst.
The reaction is carried out in the absence or presence of a solvent. It is advantageous that the solvent used can dissolve at least 0.5 % by mass, preferably at least 5 % by mass, of the above-described fluorine-containing adamantane derivative (I). The solvent may be used in such an amount as to provide a concentration of the above-described fluorine-containing adamantane derivative (I) of at least 0.5 % by mass, preferably at least 5 % by mass. The above-described fluorine-containing adamantane derivative (I) may be present in a suspended state but is preferably in a dissolved state. Specific examples of the solvent include hexane, heptane, toluene, DMF (dimethylformamide), DMAc (N,N-dimethylacetaminde), DMSO (dimethylsulfoxide), ethyl acetate, diethyl ether and tetrahydrofuran. These may be used singly or in combination of two or more thereof.
The obtained reaction product may be refined by distillation, crystallization, column separation, etc. The refining method may be suitably selected depending upon the properties of the product and the kind of impurities.

The resin composition according to the present invention comprises the above-described fluorine-containing adamantane derivative (II). As the resin composition according to the present invention, a mixed resin containing the above-described fluorine-containing adamantane derivative (II) and other polymerizable monomer and/or an epoxy resin may also used. As the "other polymerizable monomer", there may be mentioned, for example, methyl (meth)acrylate, ethyl (meth)acrylate, propyl (meth)acrylate, butyl (meth)acrylate, ethylene glycol di (meth) acrylate, 1,3-propane diol di (meth) acrylate, 1,4-butane diol di(meth)acrylate, pentaerythritol tri (meth) acrylate, pentaerythritol tetra(meth)acrylate, dipentaerythritol penta(meth)acrylate, dipentaerythritol hexa(meth)acrylate, trimethylolpropane tri(meth)acrylate, ditrimethylolpropane tetra(meth)acrylate, 1H,1H-perfluoropropyl (meth)acrylate, 1H,1H-perfluorobutyl (meth)acrylate, 1H,1H-perfluorohexyl (meth)acrylate, 1H,1H-perfluorooctyl (meth)acrylate, 1H,1H-perfluorodecyl (meth)acrylate, perfluoro-1-adamantyl (meth)acrylate, perfluoro-2-adamantyl (meth)acrylate, 1H,1H,6H,5H-perfluoro-1,5-hexane diol di(meth)acrylate, 1H,1H,8H,8H-perfluoro-1,8-octane diol di(meth)acrylate, 1H,1H,10H,10H-perfluoro-1,10-decane diol di(meth)acrylate and perfluoro-1,3-adamantane diol di(meth)acrylate. These may be used singly or in combination of two or more thereof.

As the epoxy resin, there may be mentioned, for example, glycidyl ether epoxy resins such as a bisphenol A epoxy resin, a bisphenol F epoxy resin, a bisphenol S epoxy resin, a bisphenol AD epoxy resin, a hydrogenated bisphenol A epoxy resin, a bisphenol G epoxy resin, a tetramethylbisphenol A epoxy resin, a fluorine-containing epoxy resin (e.g. bisphenol AF epoxy resin and perfluoro-1,3-bis(glycidyloxy)adamantane) and a bisphenol C epoxy resin; novolak epoxy resins such as a phenol novolak epoxy resin and a cresol novolak epoxy resin; alicyclic epoxy resins; nitrogen-containing cyclic epoxy resins such as triglycidyl isocyanurate and a hydantoin epoxy resin; aliphatic epoxy resins; glycidyl ester epoxy resins such as glycidyl (meth)acrylate; biphenyl type epoxy resins and dicyclo type epoxy resins that become a mainstream of a low water absorption curing type; naphthalene type epoxy resins; and polyfunctional epoxy resins such as trimethylolpropane polyglycidyl ether, glycerol polyglycidyl ether and pentaerythritol polyglycidyl ether. These may be used singly or in combination of two or more thereof.

The epoxy resin may be solid or liquid at ambient temperature. In general, the epoxy resin employed preferably has an average epoxy equivalent of 100 to 2,000. When the epoxy equivalent is 100 or more, a cured product of the resin composition of the present invention is not brittle but has suitable strength. When the epoxy equivalent is 2,000 or less, the glass transition point (Tg) of a cured product is not low but is in a suitable range.
In a mixed resin composed of the above-described fluorine-containing adamantane derivative (II) and the "other polymerizable monomer and/or an epoxy resin", the content of the above-described fluorine-containing adamantane derivative (II) is preferably at least 5 % by mass, more preferably at least 10 % by mass. When the content of the above-described fluorine-containing adamantane derivative (II) is at least 5 % by mass, the resin composition of the present invention can provide satisfactory optical characteristics, long-term heat resistance and electrical characteristics.

The resin composition of the present invention can be cured by polymerization using a thermal initiator and/or a photoinitiator. Any thermal initiator may be used as long as it can react with an unsaturated bond-bearing group, an epoxy group or an oxetanyl group upon heating. The thermal initiator may be, for example, an organic peroxide such as benzoyl peroxide, methyl ethyl ketone peroxide, methyl isobutyl peroxide, cumene hydroperoxide or t-butyl hydroperoxide or an azo type initiator such as azobisisobutyronitrile. These may be used singly or in combination of two or more thereof.
Any photoinitiator may be used as long as it can react with an unsaturated bond-bearing group, an epoxy group or an oxetanyl group upon light irradiation. Examples of the photoinitiator include acetophenones, benzophenones, benzyls, benzoin ethers, benzyl ketals, thioxanthones, acylphosphine oxides, acylphosphine esters, aromatic diazonium salts, aromatic sulfonium salts, aromatic iodonium salts, aromatic iodosyl salts, aromatic sulfoxonium salts and metallocene compounds. These may be used singly or in combination of two or more thereof.
The thermal initiator and/or photoinitiator may be preferably used in an amount of 0.01 to 10 parts by mass, more preferably 0.05 to 5 parts by mass, per 100 parts by mass of the above-described fluorine-containing adamantane derivative (II) or the above-described mixed resin (hereinafter occasionally referred to as resin component). When the content of the polymerization initiator is within the above range, the polymerization can proceed in a suitable manner to form a product having good optical characteristics.

If desired, the resin composition of the present invention can be compounded with a variety of customarily employed additives such as a curing accelerator, an antidegradant, a modifying agent, a silane coupling agent, a defoaming agent, an inorganic powder, a solvent, a leveling agent, a releasing agent, a dye and a pigment.
The curing accelerator is not specifically limited. Examples of the curing accelerator include tertiary amines such as 1,8-diazabicyclo[5.4.0]undecene-7, triethylenediamine and tris(2,4,6-dimethylaminomethyl)phenol, imidazoles such as 2-ethyl-4-methylimidazole and 2-methylimidazole, phosphorus compounds such as triphenylphosphine, tetraphenylphosphonium bromide, tetraphenylphosphonium tetraphenylborate and tetra-n-butylphosphonium o,o-diethylphosphorodithioate, quaternary ammonium salts, organic metal salts and derivatives of these compounds. These accelerators may be used singly or in combination of two or more thereof. Among the above curing accelerators, it is preferable to use a tertiary amine, an imidazole or a phosphorus compound.
The content of the curing accelerator is preferably 0.01 to 8.0 parts by mass, more preferably 0.1 to 3.0 parts by mass, per 100 parts by mass of the above-described resin component. When the content of the curing accelerator falls within the above range, a satisfactory curing accelerating effect can be obtained without causing coloration of the cured product.

As the antidegradant, customarily employed antidegradant may be used. Examples of the antidegradant include phenol compounds, amine compound, organic sulfur compounds and phosphorus compounds.
The phenol compound may be a commercially available product such as Irganox 1010 (manufactured by Ciba Speciality Chemicals, trademark), Irganox 1076 (manufactured by Ciba Speciality Chemicals, trademark), Irganox 1330 (manufactured by Ciba Speciality Chemicals, trademark), Irganox 3114 (manufactured by Ciba Speciality Chemicals, trademark), Irganox 3125 (manufactured by Ciba Speciality Chemicals, trademark), Irganox 3790 (manufactured by Ciba Speciality Chemicals, trademark) BHT, Cyanox 1790 (manufactured by American Cyanamide Corporation, trademark) and Sumilizer GA-80 (manufactured by Sumitomo Chemical Co., Ltd., trademark).

The amine compound may be, for example, Irgastab FS042 (manufactured by Ciba Speciality Chemicals, trademark) ; GENOX EP (manufactured by Crompton Corporation, trademark, chemical name: dialkyl-N-methylamine oxide) ; and sterically hindered amines such as ADK STAB LA-52, LA-57, LA-62, LA-63, LA-67, LA-68, LA-77, LA-82, LA-87 and LA-94 (manufactured by Adeka Corporation), Tinuvin123, 144, 440 and 662, Chimassorb 2020, 119 and 944 (manufactured by CSC), Hostavin N30 (manufactured by Hoechst Inc.), Cyasorb UV-3346 and UV-3526 (manufactured by Cytec Inc.), Uval 299 (manufactured by GLC) and Sanduvor PR-31 (manufactured by Clariant Corporation) .
The organic sulfur compound may be, for example, DSTP "YOSHITOMI" (manufactured by Yoshitomiyakuhin Co., Ltd., trademark), DLTP "YOSHITOMI" (manufactured by Yoshitomiyakuhin Co., Ltd., trademark), DLTOIB (manufactured by Yoshitomiyakuhin Co., Ltd., trademark), DMTP "YOSHITOMI" (manufactured by Yoshitomiyakuhin Co., Ltd., trademark), Seenox 412S (manufactured by Shipro Kasei Kaisha Ltd. , trademark) and Cyanox 1212 (manufactured by American Cyanamide Corporation, trademark).

As the modifying agent, customarily employed modifying agents such as glycols, silicones and alcohols may be used. As the silane coupling agent, customarily employed silane coupling agents such as silane compounds and titanates may be used. As the antifoaming agent, customarily employed antifoaming agents such as silicones may be used. The inorganic powder having a particle size of several nm to 10µm may be used depending upon the object of use. As the inorganic powder, customarily employed inorganic powder such as glass powder, silica powder, titania, zinc oxide and alumina may be used. A solvent may be used when the resin component is in the form of powder. A solvent may also be used as a diluent for coating. The solvent may be, for example, an aromatic solvent such as toluene and xylene or a ketone solvent such as methyl ethyl ketone, methyl isobutyl ketone and cyclohexanone.

The resin composition of the present invention obtainable by mixing the above resin component, thermal initiator and/or photoinitiator, and desired additives may be poured in a mold (resin mold) or coated to obtain a desired shape, and then cured by heating or irradiation of UV or the like. In the case of thermal curing, the curing temperature is generally about 30 to 200°C, preferably 50 to 150°C. A curing temperature of 30°C or higher can prevent occurrence of curing failure, while a curing temperature of 200°C or lower can prevent occurrence of coloration. The curing time is preferably 0.5 to 6 hours, though it varies with the kind of the resin component and polymerization initiator.
In the case of photo curing by irradiation of UV rays, the UV irradiation intensity is arbitrarily determined in view of the kinds of the resin component, polymerization initiator and the thickness of the intended cured product, etc. but is generally about 100 to 5,000 mJ/cm², preferably 500 to 4,000 mJ/cm². The UV irradiation may be followed by heating which is preferably carried out at 70 to 200°C for 0.5 to 12 hours.
The molding method is not specifically limited and may be, for example, injection molding, blow molding or press molding. It is, however, preferred that the molding be carried out in such a manner that the resin composition in the form of pellets is subjected to injection molding using an injection molding machine.

The cured product obtained by curing the resin composition of the present invention is excellent in heat resistance and in mechanical properties such as mar resistant property and has a low reflection index and, therefore, is useful as a low reflection layer of a reflection preventing film for a liquid crystal display and a plasma display. The reflection preventing film of the present invention may be prepared by applying the resin composition of the present invention, directly or via a layer having a different refractive index, to a substrate as such or after being diluted with a solvent, the applied coating being subsequently cured. If necessary, the solvent is evaporated before the curing.
The substrate is preferably in the form of a film or sheet and may be made of, for example, polyethylene terepthalate (PET), triacetyl cellulose (TAC), polycarbonate, polymethyl methacrylate, polyolefin or polyvinyl chloride. The solvent used for diluting the resin composition of the present invention may be, for example, an alcohol solvent such as methanol, ethanol or isopropanol, an ester solvent such as ethyl acetate, propyl acetate or butyl acetate or a ketone solvent such as methyl ethyl ketone, methyl isobutyl ketone or cyclohexanone. The resin composition of the present invention may be applied by any conventionally known coating method. Examples of such coating methods include a roll coating method, a dip coating method and a spin coating method. A curing method for the formation of the reflection preventing film is preferably the above-described photo curing method.

The resin composition of the present invention is suitably used for forming optical semiconductors (LED, etc.), as a resin (sealing material or adhesive agent) for electric circuits and optical circuits (optical waveguides), and for forming optoelectric parts such as optical communication lenses and optical films.
Thus, the resin composition of the present invention may be used for forming semiconductor elements or integrated circuits (IC, etc.), discrete semiconductors (diodes, transistors, thermisters), LED (LED lamps, chip LED, light receiving elements, optical semiconductor lenses), sensors (temperature sensors, optical sensors, magnetic sensors), passive components (high frequency devices, resistors, capacitors, etc.), electromechanical components (connectors, switches, relays, etc.), automobile parts (circuit systems, control systems, sensors, lamp seals, etc.), adhesives (optical parts, optical discs, pickup lenses) and surface coatings (optical films).

Further, a cured product obtained by curing the resin composition of the present invention may be suited for use as, for example, a coating agent, a liquid crystal spacer, a reflection preventing film for semiconductor resists, a material for nanoinprint, an optical fiber, an optical waveguide, a lens such as a Fresnel lens, a lenticular lens or a microlens array, and a refractive index modulation material for volume holograms.

### EXAMPLES

The present invention will be next described in more detail by way of examples but is not limited thereto in any way.

### Example 1

### Synthesis of Perfluoro-1,3-adamantanedimethanol:

In a reaction vessel having an inside volume of 10 L and equipped with a condenser, a NaF pellet packed layer and a thermometer, 5.0 L of 1,1,2-trichlorotrifluoroethane were placed and maintained at an inside temperature of 0°C. Nitrogen and fluorine gas were then blown into the vessel at flow rates of 2,000 mL/min and 630 mL/min, respectively. A solution of 100 g of diethyl adamantanedicarboxylate dissolved in 1.0 L of 1,1,2-trichlorotrifluoroethane was added dropwise over 24 hours to the vessel 3 minutes after the start of the gas feed.
Then, after changing the flow rates of the nitrogen and fluorine gases to 1,200 mL/min and 300 mL/min, respectively, a solution of 4 g of benzene dissolved in 30 mL of 1,1,2-trichlorotrifluoroethane was added dropwise over 30 minutes. The reaction mixture was stirred for 15 minutes to terminate the reaction. The feed of the fluorine gas was stopped and the solvent was removed by distillation to obtain a perfluorinated compound of diethyl adamantanedicarboxylate.
In a four-necked flask having an inside volume of 2,000 mL and equipped with a stirrer, a condenser and a thermometer, 33.7 g of sodium borohydride and 500 mL of diglime were placed, to which a solution of the above perfluorinated compound dissolved in 200 g of Halocarbon oil 0.8 was added dropwise while maintaining the inside temperature not exceeding 35°C. The inside temperature was then raised to 60°C and the reaction mixture was stirred for 4 hours. The reaction liquid was then allowed to cool to room temperature and added slowly with 500 mL of pure water. This was made acidic by addition of a small amount of hydrochloric acid. The organic layer was separated and washed twice with 500 mL of 1 mol/L hydrochloric acid and once with 500 mL of pure water. The solvent was then removed by distillation to obtain perfluoro-1,3-adamantanedimethanol (yield: 75 %, purity by GC: 97.5 %) as the desired product.
Perfluoro-1,3-adamantanedimethanol thus obtained was identified by nuclear magnetic resonance spectra (¹H-NMR, ¹³C-NMR, ¹⁹F-NMR) and by GC-MS. The obtained spectrum data are as follows.
The nuclear magnetic resonance spectra were measured with JNM-ECA500 manufactured by JEOL Ltd. using DMSO-d₆ as a solvent. GC-MS was measured with GCMS-QP2010 manufactured by Shimadzu Corporation.

¹H-NMR (500 MHz): 4.42 (d, 4H), 6.05 (t, 2H) ¹³C-NMR (125 MHz): 53.0
¹⁹F-NMR (465 MHz): -107.7, -115.9, -123.0, -221.0 (values determined using α,α,α-trifluorotoluene as a standard substance which is assigned -64.0) GC-MS (EI): 448 (M⁺, 0.5 %), 418 (7.2 %), 181 (10.9 %), 145 (12.7 %), 31 (100 %)

### Example 2

### Synthesis of Perfluoro-1,3-bis(acryloyloxymethyl)adamantane:

In a four-necked flask having an inside volume of 1,000 mL and equipped with a stirrer, a thermometer, a reflux condenser and a dropping funnel, 50.0 g of perfluoro-1,3-adamantanedimethanol obtained in Example 1 were placed and dissolved in 500 mL of chloroform. This was added with 51 mL of triethylamine and then dropwise with 30 mL of acryloyl chloride from the dropping funnel while maintaining the reaction system at a temperature not exceeding 25°C. After completion of the dropwise addition, the reaction mixture was stirred at room temperature for 1 hour. Then, the mixture was added with 250 mL of a 5 % by mass aqueous sodium chloride solution and stirred for 10 minutes. The chloroform layer was separated and washed twice with 250 mL of a 5 % by mass aqueous sodium chloride solution. The chloroform layer was separated and dehydrated with anhydrous magnesium sulfate. Using an evaporator, chloroform was distilled off to obtain a crude product. The crude product was dissolved in 500 mL of toluene, to which 12.4 g of silica gel were added. The mixture was stirred for 30 minutes for decolorization. The silica gel was removed by filtration and toluene was distilled off using an evaporator to obtain perfluoro-1,3-bis(acryloyloxymethyl)adamantane represented by the formula shown below (yield: 85 %, purity by GC: 98.4 %).

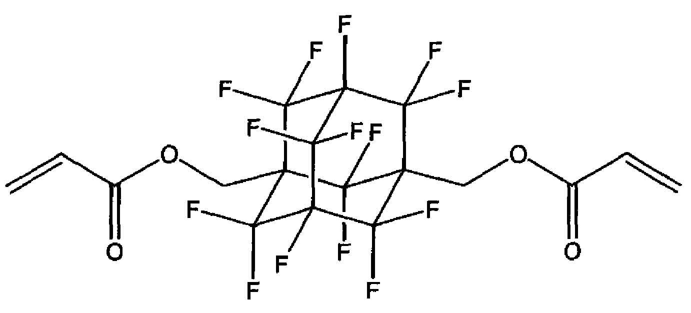

Perfluoro-1,3-bis(acryloyloxymethyl)adamantane thus obtained was identified by nuclear magnetic resonance spectra (¹H-NMR, ¹³C-NMR, ¹⁹F-NMR) and by GC-MS. The obtained spectrum data are as follows. The nuclear magnetic resonance spectra were measured with JNM-ECA500 manufactured by JEOL Ltd. using chloroform-d as a solvent. GC-MS was measured with GCMS-QP2010 manufactured by Shimadzu Corporation.

¹H-NMR (500 MHz) : 5.06 (s, 4H), 5.97 (d, 2H), 6.15 (dd, 2H), 6.48 (d, 2H)
¹³C-NMR (125 MHz): 53.0, 126.9, 133.2, 164.3
¹⁹F-NMR (465 MHz):-105.1, -113.9, -121.7, -219.4 (values
determined using α,α,α-trifluorotoluene as a standard substance which is assigned -64.0)
GC-MS (EI): 556 (M⁺, 2.1 %), 501 (2.0 %), 85 (0.7 %), 55 (100 %)

### Example 3

### Synthesis of Perfluoro-1,3-bis(methacryloyloxymethyl)adamantane:

The procedures of Example 2 were carried out in the same manner as described except that 36 mL of methacryloyl chloride were used in place of 30 mL of acryloyl chloride used in Example 2 to obtain perfluoro-1,3-bis(methacryloyloxymethyl)adamantane represented by the formula shown below (yield: 83 %, purity by GC: 98.7 %).

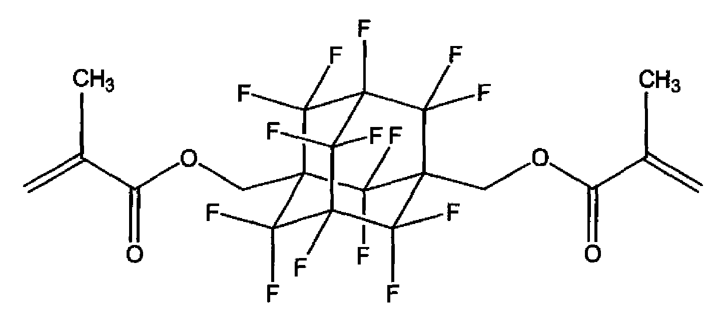

Perfluoro-1,3-bis(methacryloyloxymethyl)adamantane thus obtained was identified in the same manner as that in Example 2. The obtained spectrum data are as follows.

¹H-NMR (500 MHz): 1.96 (s, 6H), 5.03 (s, 4H), 5.69 (s, 2H), 6.18 (s, 2H)
¹³C-NMR (125 MHz): 18.0, 53.4, 128.0, 134.9, 165.7
¹⁹F-NMR (465MHz): -105.1, -113.9, -121.6, -219.4 (values determined using α,α,α-trifluorotoluene as a standard
substance which is assigned -64.0)
GC-MS (EI) : 584 (M⁺, 9.9 %), 515 (4.3 %), 69 (100 %), 41 (42.1 %)

### Example 4

### Synthesis of 2,2'-(Perfluoroadamantane-1,3-diyl)bis(2,2-difluoroethanol):

The reaction procedures of Example 1 were carried out in the same manner as described except that 100 g of dibutyl adamantane-1,3-diacetate were used in place of 100 g of diethyl adamantanedicarboxylate used in Example 1 and that the using amount of sodium borohydride was changed to 25.9 g. 2,2'-(Perfluoroadamantane-1,3-diyl)bis(2,2-difluoroethanol) thus obtained was purified by recrystallization from a toluene/heptane mixed solution (yield: 82 %, purity by GC: 98.5 %).
2,2'-(Perfluoroadamantane-1,3-diyl)-bis(2,2-difluoroethan ol) thus obtained was identified by nuclear magnetic resonance spectra (¹H-NMR, ¹³C-NMR, ¹⁹F-NMR) and by GC-MS. The obtained spectrum data are as follows. The nuclear magnetic resonance spectra were measured with JNM-ECA500 manufactured by JEOL Ltd. using DMSO-d₆ as a solvent. GC-MS was measured with GCMS-QP2010 manufactured by Shimadzu Corporation.

¹H-NMR (500 MHz): 4.05 (tt, 4H), 6.23 (t, 2H)
¹³C-NMR (125 MHz): 63.0
¹⁹F-NMR (465 MHz): -97.4, -101.6, -111.5, -123.1, -219.7 (values determined using α,α,α-trifluorotoluene as a standard substance which is assigned -64.0)
GC-MS (EI): 517 (M⁺, 8.4 %), 81 (9.5 %), 62 (37.2 %), 31(100 %)

### Example 5

### Synthesis of 2,2'-(Perfluoroadamantane-1,3-diyl)bis(2,2-difluoroethanol) diacrylate:

In a four-necked flask having an inside volume of 1,000 mL and equipped with a stirrer, a thermometer, a reflux condenser and a dropping funnel, 50.0 g of 2,2'-(perfluoroadamantane-1,3-diyl)-bis(2,2-difluoroethanol) obtained in Example 4 were placed and dissolved in 250 mL of chloroform. This was added with 38 mL of triethylamine and then dropwise with 22 mL of acryloyl chloride from the dropping funnel while maintaining the reaction system at a temperature not exceeding 25°C. After completion of the dropwise addition, the reaction mixture was stirred at room temperature for 1 hour. Then, the mixture was added with 250 mL of chloroform and then 250 mL of a 5 % by mass aqueous sodium chloride solution and stirred for 10 minutes. The chloroform layer was separated and washed twice with 250 mL of a 5 % by mass aqueous sodium chloride solution. The chloroform layer was separated and dehydrated with anhydrous magnesium sulfate. Using an evaporator, chloroform was distilled off to obtain a crude product. The crude product was dissolved in 500 mL of n-hexane, to which 5 g of silica gel were added. The mixture was stirred for 30 minutes for decolorization. The silica gel was removed by filtration and n-hexane was distilled off using an evaporator to obtain 2,2'-(perfluoroadamantane-1,3-diyl)-bis(2,2-difluoroethanol) diacrylate represented by the formula shown below (yield: 76 %, purity by GC: 99.5 %).

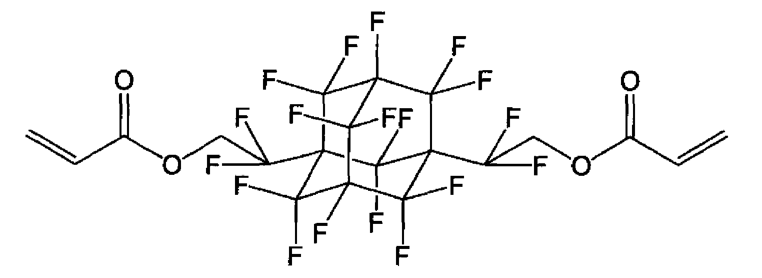

2,2'-(Perfluoroadamantane-1,3-diyl)-bis(2,2-difluoroethan ol) diacrylate thus obtained was identified by nuclear magnetic resonance spectra (¹H-NMR, ¹³C-NMR, ¹⁹F-NMR) and by GC-MS. The obtained spectrum data are as follows. The nuclear magnetic resonance spectra were measured with JNM-ECA500 manufactured by JEOL Ltd. using CDCl₃ as a solvent. GC-MS was measured with GCMS-QP2010 manufactured by Shimadzu Corporation.

¹H-NMR (500 MHz) : 4.81 (t, 4H), 5.98 (d, 2H), 6.19 (dd, 2H), 6.53 (d, 2H)
¹³C-NMR (125 MHz): 63.0, 126.9, 133.2, 164.6
¹⁹F-NMR (465 MHz): -96.1, -98.7, -110.0, -121.8, -218.3 (values determined using α,α,α-trifluorotoluene as a standard substance which is assigned -64.0)
GC-MS (EI): 655 (M⁺, 2.4 %), 547 (4.5 %), 85 (3.3 %), 55 (100 %)

### Example 6

### Synthesis of Perfluoro-1,3,5-adamantanetrimethanol:

The reaction procedures of Example 1 were carried out in the same manner as described except that 100 g of dibutyl adamantane-1,3,5-tricarboxylate were used in place of 100 g of diethyl adamantanedicarboxylate used in Example 1 and that the using amount of sodium borohydride was changed to 30.3 g. Perfluoro-1,3,5-adamantanetrimethanol thus obtained was purified by recrystallization from a toluene/heptane mixed solution (yield: 57 %, purity by GC: 99.1 %).
Perfluoro-1,3,5-adamantanetrimethanol thus obtained was identified by nuclear magnetic resonance spectra (¹H-NMR, ¹³C-NMR, ¹⁹F-NMR) and by GC-MS. The obtained spectrum data are as follows. The nuclear magnetic resonance spectra were measured with JNM-ECA500 manufactured by JEOL Ltd. using DMSO-d₆ as a solvent. GC-MS was measured with GCMS-QP2010 manufactured by Shimadzu Corporation.

¹H-NMR (500 MHz): 4.29 (d, 6H), 5.71 (t, 3H)
¹³C-NMR (125 MHz): 53.5
¹⁹F-NMR (465 MHz) : -107.0, -115.3, -223.2 (values determined using α,α,α-trifluorotoluene as a standard substance which is assigned -64.0)
GC-MS (EI): 460 (M⁺, 0.4 %), 430 (12.2 %), 31 (100 %)

### Example 7

### Synthesis of Perfluoro-1,3,5-tris(acryloyloxymethyl)adamantane:

In a four-necked flask having an inside volume of 200 mL and equipped with a stirrer, a thermometer, a reflux condenser having a Dean-Stark trap and a three-way cock, 30.0 g of perfluoro-1,3,5-adamantanetrimethanol obtained in Example 6, 18.5 g of acrylic acid, 2.1 g of 98 % by mass sulfuric acid, 18 mg of methoquinone and 75 mL of toluene were charged. The flask was immersed in an oil bath at 130°C. Air was streamed in a small amount through the three-way cock and stirring was started. The reaction was continued for 8 hours after toluene began refluxing. The reaction liquid was then allowed to cool to room temperature, added with 90 mL of toluene and transferred to a separatory funnel. The toluene layer was washed once with 150 mL of a 3 % by mass aqueous disodium hydrogen phosphate solution, once with 150 mL of a 1 % by mass aqueous sodium phosphate solution, and once with 150 mL of a 5 % by mass aqueous sodium chloride solution. The toluene layer was separated and dehydrated with anhydrous magnesium sulfate. The anhydrous magnesium sulfate was removed by filtration. The toluene layer was mixed with 10 g of silica gel and the mixture was stirred for 30 minutes for decolorization. The silica gel was removed by filtration and toluene was distilled off using an evaporator to obtain perfluoro-1,3,5-tris(acryloyloxymethyl)adamantane represented by the formula shown below (yield: 78 %, purity by GC: 98.2 %).

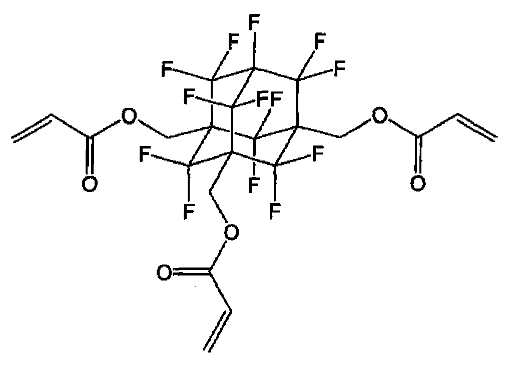

Perfluoro-1,3,5-tris(acryloxxymethyl)adamantane thus obtained was identified by nuclear magnetic resonance spectra (¹H-NMR, ¹³C-NMR, ¹⁹F-NMR) and by GC-MS. The obtained spectrum data are as follows. The nuclear magnetic resonance spectra were measured with JNM-ECA500 manufactured by JEOL Ltd. using CDCl₃ as a solvent. GC-MS was measured with GCMS-QP2010 manufactured by Shimadzu Corporation.

¹H-NMR (500 MHz): 5.04 (s, 6H), 5.96 (d, 3H), 6.14 (dd, 3H), 6.47 (d, 3H)
¹³C-NMR (125 MHz): 53.2, 127.0, 132.8, 164.4
¹⁹F-NMR (465 MHz) : -104.7, -113.5, -221.5 (values determined using α,α,α-trifluorotoluene as a standard substance which is assigned -64.0)
GC-MS (EI): 622 (M⁺, 2.9 %), 567 (2.6 %), 85 (0.7 %), 55 (100 %)

### Example 8

### Synthesis of Perfluoro-1-adamantanemethanol:

The reaction procedures of Example 1 were carried out in the same manner as described except that 100 g of ethyl adamantane-1-carboxylate were used in place of 100 g of diethyl adamantanedicarboxylate used in Example 1 and that the using amount of sodium borohydride was changed to 23.6 g. Perfluoro-1-adamantanemethanol thus obtained was purified by recrystallization from a toluene/heptane mixed solution (yield: 84 %, purity by GC: 98.0 %).
Perfluoro-1-adamantanemethanol thus obtained was identified by nuclear magnetic resonance spectra (¹H-NMR, ¹³C-NMR, ¹⁹F-NMR) and by GC-MS. The obtained spectrum data are as follows. The nuclear magnetic resonance spectra were measured with JNM-ECA500 manufactured by JEOL Ltd. using DMSO-d₆ as a solvent. GC-MS was measured with GCMS-QP2010 manufactured by Shimadzu Corporation.

¹H-NMR (500 MHz): 4.55 (d, 2H), 6.37 (t, 1H)
¹³C-NMR (125 MHz): 53.0
¹⁹F-NMR (465 MHz) : -116.1, -123.2, -221.3 (values determined using α,α,α-trifluorotoluene as a standard substance which is assigned -64.0)
GC-MS (EI): 397 (M⁺, 1.6 %), 131 (6.5 %), 61 (5.9 %), 31 (100 %)

### Example 9

### Synthesis of Perfluoro-1-(acryloyloxymethyl)adamantane:

In a four-necked flask having an inside volume of 500 mL and equipped with a stirrer, a thermometer, a reflux condenser and a dropping funnel, 30.0 g of perfluoro-1-adamantanemethanol obtained in Example 8 were placed and dissolved in 150 mL of chloroform. This was added with 19 mL of triethylamine and then dropwise with 11 mL of acryloyl chloride from the dropping funnel while maintaining the reaction system at a temperature not exceeding 25°C. After completion of the dropwise addition, the reaction mixture was stirred at room temperature for 1 hour. Then, the mixture was added with 150 mL of chloroform and then 150 mL of a 5 % by mass aqueous sodium chloride solution and stirred for 10 minutes. The chloroform layer was separated and washed twice with 150 mL of a 5 % by mass aqueous sodium chloride solution. The chloroform layer was separated and dehydrated with anhydrous magnesium sulfate. Using an evaporator, chloroform was distilled off to obtain a crude product. The crude product was dissolved in 300 mL of n-hexane, to which 3 g of silica gel were added. The mixture was stirred for 30 minutes for decolorization. The silica gel was removed by filtration and n-hexane was distilled off using an evaporator to obtain perfluoro-1-(acryloyloxymethyl)adamantane represented by the formula shown below (yield: 84 %, purity by GC: 99.0 %).

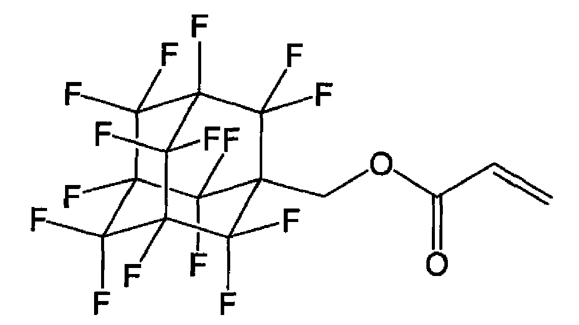

Perfluoro-1-(acryloyloxymethyl)adamantane thus obtained was identified by nuclear magnetic resonance spectra (¹H-NMR, ¹³C-NMR, ¹⁹F-NMR) and by GC-MS. The obtained spectrum data are as follows. The nuclear magnetic resonance spectra were measured with JNM-ECA500 manufactured by JEOL Ltd. using CDCl₃ as a solvent. GC-MS was measured with GCMS-QP2010 manufactured by Shimadzu Corporation.

¹H-NMR (500 MHz): 5.09 (s, 2H), 5.98 (d, 1H), 6.15 (dd, 1H), 6.49 (d, 1H)
¹³C-NMR (125 MHz): 52.6, 126.7, 133.3, 164.1
¹⁹F-NMR (465 MHz): -114.1, -121.8, -220.1 (values determined using α,α,α-trifluorotoluene as a standard substance which is assigned -64.0) GC-MS (EI) : 490 (M⁺, 5.0 %), 55 (100 %), 27 (20.7 %)

### Example 10

### Synthesis of Perfluoro-1-(methacryloyloxymethyl)adamantane:

The procedures of Example 9 were carried out in the same manner as described except that 13 mL of methacryloyl chloride were used in place of 11 mL of acryloyl chloride used in Example 9 to obtain perfluoro-1-(methacryloyloxymethyl)adamantane represented by the formula shown below (yield: 75 %, purity by GC: 99.1 %).

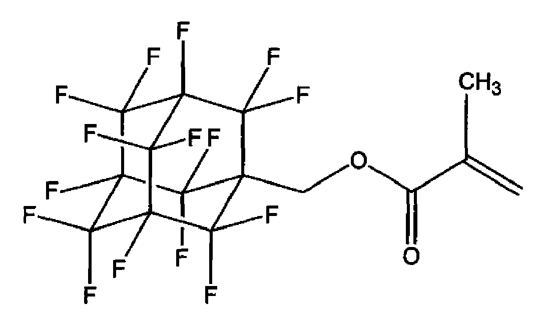

Perfluoro-1-(methacryloyloxymethyl)adamantane thus obtained was identified by nuclear magnetic resonance spectra (¹H-NMR, ¹³C-NMR, ¹⁹F-NMR) and by GC-MS. The obtained spectrum data are as follows. The nuclear magnetic resonance spectra were measured with JNM-ECA500 manufactured by JEOL Ltd. using CDCl₃ as a solvent. GC-MS was measured with GCMS-QP2010 manufactured by Shimadzu Corporation.

¹H-NMR (500 MHz): 1.94 (s, 3H), 5.04 (s, 2H), 5.69 (s, 1H), 6.17 (s, 1H)
¹³C-NMR (125 MHz): 10.5, 45.6, 120.7, 127.3, 158.1
¹⁹F-NMR (465 MHz): -114.5, -122.2, -220.7 (values determined using α,α,α-trifluorotoluene as a standard substance which is assigned -64.0)
GC-MS (EI): 504 (M⁺, 19.2 %), 200 (1.7 %), 145 (2. 5 %), 131 (5.8 %), 99 (5.8 %), 69 (100 %)

### Example 11

### Synthesis of 2,2'-(Perfluoroadamantane-1,3-diyl)bis(2,2-difluoroethanol) diglycidyl ether:

In a four-necked flask having an inside volume of 500 mL and equipped with a stirrer, a thermometer, a reflux condenser having a Dean-Stark trap and a dropping funnel, 25.0 g of 2,2'-(perfluoroadamantane-1,3-diyl)bis(2,2-difluoroethanol) obtained in Example 4, 67.5 g of epichlorohydrin, 2.5 g of tetraethylammonium bromide and 125 mL of toluene were charged. The flask was immersed in an oil bath at 130°C and stirring was started. After toluene had begun refluxing, 13.7 g of a 50 % by mass aqueous sodium hydroxide solution was added dropwise from the dropping funnel over 30 minutes.
The reaction was carried out for 4 hours while removing water discharged by refluxing. The reaction liquid was allowed to cool to room temperature, added with 250 mL of toluene, transferred to a separatory funnel, and washed once with 200 mL of pure water, once with 200 mL of 0.1 mol/L hydrochloric acid and further twice with 200 mL of pure water. The toluene layer was separated. The toluene was distilled off using an evaporator to obtain 2,2'-(perfluoroadamantane-1,3-diyl)bis(2,2-difluoroethanol) diglycidyl ether (yield: 90 %, epoxy equivalent: 369).

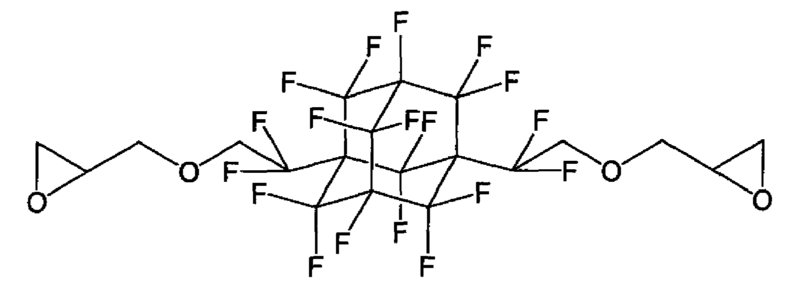

2,2'-(Perfluoroadamantane-1,3-diyl)bis(2,2-difluoroethano 1) diglycidyl ether thus obtained was identified by nuclear magnetic resonance spectra (¹H-NMR, ¹³C-NMR, ¹⁹F-NMR) and by GC-MS. The obtained spectrum data are as follows. The nuclear magnetic resonance spectra were measured with JNM-ECA500 manufactured by JEOL Ltd. using CDCl₃ as a solvent. GC-MS was measured with GCMS-QP2010 manufactured by Shimadzu Corporation.

¹H-NMR (500 MHz): 2.63 (dd, 2H), 2.81 (dd, 2H), 3.18 (m, 2H), 3.56 (dd, 2H), 4.00 (dd, 2H), 4.14 (m, 4H)
¹³C-NMR (125 MHz): 43.7, 50.5, 72.0, 73.7
¹⁹F-NMR (465 MHz): -96.2, -99.6, -110.1, -121.8, -218.5 (values determined using α,α,α-trifluorotoluene as a standard substance which is assigned -64.0) GC-MS (EI) : 587 (M⁺, 13.5 %), 87 (19.9 %), 57 (100 %), 43 (16.3 %), 29 (93.1 %)

### Example 12

To 100 parts by mass of 2,2'-(perfluoroadamantane-1,3-diyl)bis-(2,2-difluoroethanol) diacrylate obtained in Example 5 were added 2 parts by mass of benzoin isobutyl ether as a photopolymerization initiator. After thorough mixing, the mixture was deaerated under vacuum to obtain a resin composition. The resin composition was poured in a glass cell and irradiated with UV rays using a mercury lamp at an intensity of 1,000 mJ/cm² to obtain a cured product having a thickness of 1 mm. The cured product was evaluated by the methods described below. The results are summarized in Table 1.

(1) Thermal decomposition temperature
   A sample (10 mg) was heated at a rate of 10°C/min in the atmosphere of nitrogen using a simultaneous differential thermal and thermogravimetric analyzer (TG/DTA 6200 manufactured by Seiko Instruments Co., Ltd.) to measure a temperature (Td1) at which the weight thereof was reduced by 1 % by mass.
(2) Durometer hardness D
   Durometer hardness was measured in accordance with JIS K7215 using Durometer D (manufactured by Shore Inc.) as a measuring device.
(3) Bending test
   Bending test was performed in accordance with JIS K7171 using Universal Testing Machine (Model 5582 manufactured by Instron Inc.) as a measuring device.
(4) Refractive index
   Refractive index was measured at 23°C using Abbe refractometer manufactured by Atago Co., Ltd.

### Example 13

A cured product was prepared and evaluated in the same manner as described in Example 12 except that perfluoro-1,3,5-tris(acryloyloxymethyl)adamantane obtained in Example 7 was used in place of 2,2'-(perfluoroadamantane-1,3-diyl)bis(2,2- difluoroethanol) diacrylate used in Example 12. The evaluation results are shown in Table 1.

### Example 14

A cured product was prepared and evaluated in the same manner as described in Example 12 except that 100 parts by mass of 2,2'-(perfluoroadamantane-1,3-diyl)bis(2,2-difluoroethanol) diacrylate used in Example 12 were replaced with a mixture of 50 parts by mass of perfluoro-1,3-bis(acryloyloxymethyl)adamantane obtained in Example 2 and 50 % by mass of 1H,1H,6H,6H-perfluoro-1,6-hexanediol diacrylate. The evaluation results are shown in Table 1.

### Example 15

A cured product was prepared and evaluated in the same manner as described in Example 12 except that 100 parts by mass of 2,2'-(perfluoroadamantane-1,3-diyl)bis(2,2-difluoroethanol) diacrylate used in Example 12 were replaced with a mixture of 50 parts by mass of perfluoro-1,3,5-tris-(acryloyloxymethyl)adamantane obtained in Example 7 and 50 parts by mass of perfluoro-1-(acryloyloxymethyl)adamantane. The evaluation results are shown in Table 1.

### Example 16

To 100 pars by mass of 2,2'-(perfluoroadamantane-1,3-diyl)bis-(2,2-difluoroethanol) diglycidyl ether obtained in Example 11 were added 2 parts by mass of Irgacure 250 (manufactured by Ciba Speciality Chemicals, Inc.) as a photopolymerization initiator. After thorough mixing, the mixture was deaerated under vacuum to obtain a resin composition. The resin composition was poured in a glass cell and irradiated with UV rays using a mercury lamp at an intensity of 1,000 mJ/cm² to obtain a cured product having a thickness of 1 mm. The cured product was evaluated by the methods described above. The results are summarized in Table 1.

### Comparative Example 1

A cured product was prepared and evaluated in the same manner as described in Example 12 except that 2,2'-(perfluoroadamantane-1,3-diyl)bis(2,2-difluoroethanol) diacrylate was replaced with 1H,1H,6H,6H-perfluoro-1,6-hexanediol diacrylate. The evaluation results are shown in Table 1.

**Table 1**

| | Thermal decomposition temperature (°C) | Durometer hardness D (degree) | Bending Strength (MPa) | Bending Modulus (MPa) | Refractive Index (n_{D}²³) |
|---|---|---|---|---|---|
| Example 12 | 186 | 89 | 86.4 | 3020 | 1.427 |
| Example 13 | 180 | 91 | 56.7 | 3970 | 1.439 |
| Example 14 | 174 | 86 | 53.2 | 2880 | 1.431 |
| Example 15 | 181 | 88 | 51.9 | 2710 | 1.419 |
| Example 16 | 169 | 87 | 79.3 | 3990 | 1.433 |
| Comparative Example 1 | 143 | 82 | 32.8 | 1760 | 1.434 |

### Example 17

A coating composition for forming a hard coat layer was prepared by mixing 30 parts by mass of pentaerythritol tetraacrylate, 0.6 part by mass of Irgacure 907 (manufactured by Ciba Speciality Chemicals, Inc.) and 70 parts by mass of methyl isobutyl ketone.
Also prepared was a coating composition I for forming a low refractive layer by mixing 9.5 parts by mass of 2,2'-(perfluoroadamantane-1,3-diyl)bis(2,2-difluoroethanol) diacrylate obtained in Example 5, 0.5 part by mass of pentaerythritol tetraacrylate, 0.2 part by mass of Irgacure 907 and 90 parts by mass of methyl isobutyl ketone.
The above-obtained coating composition for forming a hard coat layer was applied onto a polyethylene terephthalate film (A4100 manufactured by Toyo Boseki Co., Ltd.) by bar coating to a thickness of 5 µm. After the solvent was evaporated to dryness, the coating was irradiated with UV rays at an intensity of 1,000 mJ/cm² using a mercury lamp and cured to form a hard coat layer. Onto the thus formed hard coat layer, the above-obtained coating composition I for forming a low refractive layer was applied by bar coating to a thickness of 0.1 µm. After the solvent was evaporated to dryness, the coating was irradiated with UV rays at an intensity of 1,000 mJ/cm² using a mercury lamp and cured to obtain a reflection preventing film. The obtained reflection preventing film was measured for its minimum reflectance and evaluated for its mar resistance in the manner described below. The evaluation results are shown in Table 2.

(1) Measurement of minimum reflectance
   A reflectance was measured using a spectrophotometer equipped with a 5-degree regular reflection measuring device (UV-3100PC manufactured by Shimadzu Corporation). The reflectance is a value measured with a wavelength of 550 nm.
(2) Evaluation of mar resistance
   On a surface of the reflection preventing film, steel wool (grade #0000) was reciprocated 10 times with an applied friction load of 250 g. The presence or absence of scratches was checked. The scratch resistance was evaluated as follows:○: No scratches were observed;×: Scratches were observed.

### Comparative Example 2

A coating composition II for forming a low refractive layer was prepared by mixing 9.5 parts by mass of 1H, 1H, 6H, 6H-perfluoro-1,6-hexanediol diacrylate, 0.5 part by mass of pentaerythritol tetraacrylate, 0.2 part by mass of Irgacure 907 and 90 parts by mass of methyl isobutyl ketone.
Example 17 was repeated in the same manner as described except that the above-obtained coating composition II for forming a low refractive layer was substituted for the coating composition I for forming a low refractive layer used in Example 17 to obtain a reflection preventing film. The obtained reflection preventing film was measured for its minimum reflectance and evaluated for its mar resistance in the manner described above. The evaluation results are shown in Table 2.

**Table 2**

| | Reflactance (%) | Mar resistance |
|---|---|---|
| Example 17 | 1.1 | ○ |
| Comparative Example 2 | 1.1 | × |

### INDUSTRIAL APPLICABILITY

The polymerizable group-containing and fluorine-containing adamantane derivative according to the present invention and the resin composition containing such a derivative have good heat resistance and good mechanical properties such as mar resistance and can give a cured product having a low refractive index. Therefore, they may be suitably used for forming a low refractive index layer for a reflection preventing film, an optical fiber, an optical waveguide and various types of lenses. Further, when they are used for forming a low refractive index layer of a reflection preventing film for a display such as an organic EL element or a liquid crystal, it is possible to improve the surface hardness of the reflection preventing film.
Further, the polymerizable group-containing and fluorine-containing adamantane derivative of the present invention, which can give a cured product having a low refractive index and good heat resistance, may be suited for use as an optical fiber or an optical waveguide material.
In addition, the polymerizable group-containing and fluorine-containing adamantane derivative of the present invention, which can give a cured product having a low refractive index and good heat resistance, may be suitably used as a reflection preventing film material of a reflection preventing film for a semiconductor resist and as a refractive index modulation material for a volume hologram.

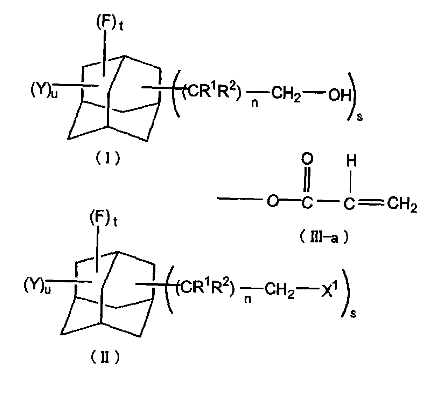

## Claims

1. A fluorine-containing adamantane derivative represented by the following general formula (I): 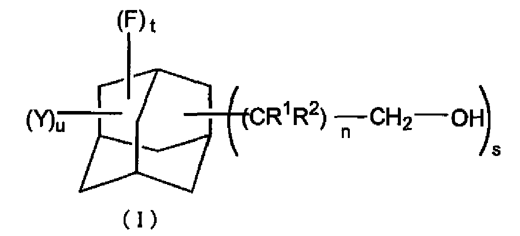 [wherein R¹ and R² each independently represent a hydrogen atom, a halogen atom or a C₁ to C₂₀ aliphatic hydrocarbon group which may contain a heteroatom or heteroatoms, n represents an integer of 0 or more, Y represents a group selected from a hydrogen atom, a hydrocarbon group, a halogen-substituted hydrocarbon group, a cyclic hydrocarbon group, a halogen-substituted cyclic hydrocarbon group, a hydroxyl group, a carboxyl group and =O formed by two Y's taken together, s and t each represent an integer of 1 to 15 and u represents an integer of 0 to 14 with the proviso that a sum of s, t and u is equal to 16].

2. A polymerizable group-containing and fluorine-containing adamantane derivative represented by the following general formula (II): 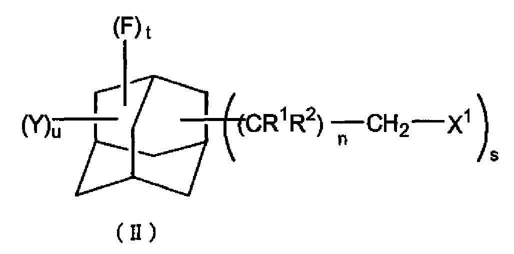 [wherein R¹ and R² each independently represent a hydrogen atom, a halogen atom or a C₁ to C₂₀ aliphatic hydrocarbon group which may contain a heteroatom or heteroatoms, n represents an integer of 0 or more, X¹ represents a polymerizable group represented by the following general formula (III), the following formula (IV) or the following general formula (V): 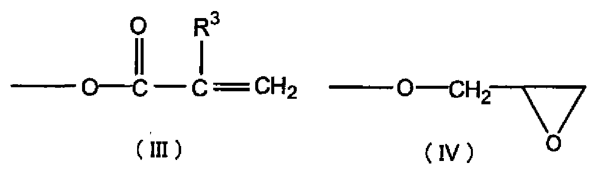 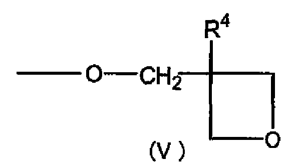 (where R³ represents a hydrogen atom, a methyl group or a trifluoromethyl group and R⁴ represents a C₁ to C₅ hydrocarbon group)
Y represents a group selected from a hydrogen atom, a hydrocarbon group, a halogen-substituted hydrocarbon group, a cyclic hydrocarbon group, a halogen-substituted cyclic hydrocarbon group, a hydroxyl group, a carboxyl group and =O formed by two Y's taken together, s and t each represent an integer of 1 to 15 and u represents an integer of 0 to 14 with the proviso that a sum of s, t and u is equal to 16].

3. A process for producing a polymerizable group-containing and fluorine-containing adamantane derivative represented by the general formula (II) shown below, comprising reacting a fluorine containing adamantane derivative represented by the general formula (I) shown below with a compound containing a polymerizable group: 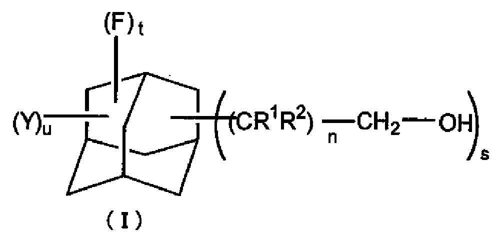 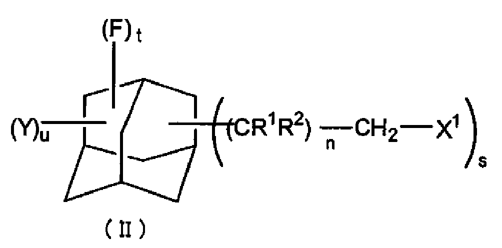 [wherein R¹ and R² each independently represent a hydrogen atom, a halogen atom or a C₁ to C₂₀ aliphatic hydrocarbon group which may contain a heteroatom or heteroatoms, n represents an integer of 0 or more, X¹ represents a polymerizable group represented by the following general formula (III), the following formula (IV) or the following general formula (V): 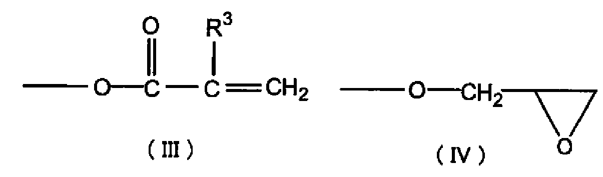 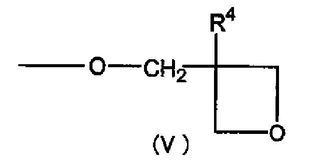 (where R³ represents a hydrogen atom, a methyl group or a trifluoromethyl group and R⁴ represents a C₁ to C₅ hydrocarbon group)
Y represents a group selected from a hydrogen atom, a hydrocarbon group, a halogen-substituted hydrocarbon group, a cyclic hydrocarbon group, a halogen-substituted cyclic hydrocarbon group, a hydroxyl group, a carboxyl group and =O formed by two Y's taken together, s and t each represent an integer of 1 to 15 and u represents an integer of 0 to 14 with the proviso that a sum of s, t and u is equal to 16].

4. A resin composition comprising a polymerizable group-containing and fluorine-containing adamantane derivative according to claim 2.

5. A reflection preventing film having a layer obtained by curing a resin composition according to claim 4.
